# EUROPEAN PATENT APPLICATION

(11) **EP 3 434 675 A1**
(43) Date of publication of application: **30.01.2019**
(21) Application number: 17769416.3
(22) Date of filing: 21.03.2017
(51) Int. Cl.: C07D 471/04, A61K 31/437, A61P 19/02

(54) **HYDROCHLORIDE SALT CRYSTAL OF DRUG FOR TREATING OR PREVENTING JAK-ASSOCIATED DISEASE AND PREPARATION METHOD THEREOF**

(30) Priority: 21.03.2016 CN 201610160274
(71) Applicant: Crystal Pharmatech Co., Ltd, Suzhou, Jiangsu 215123 (CN)
(72) Inventor: CHEN, Minhua, Suzhou Jiangsu 215123 (CN); ZHANG, Yanfeng, Suzhou Jiangsu 215123 (CN); LIU, Kai, Suzhou Jiangsu 215123 (CN); ZHANG, Xiaoyu, Suzhou Jiangsu 215123 (CN)
(74) Representative: Grünecker Patent- und Rechtsanwälte PartG mbB
(86) International application number: PCT/CN2017/077492
(87) International publication number: WO 2017/162139

(57) **Abstract**

The present disclosure disclosed crystalline forms of Filgotinib hydrochloride for treating or preventing JAK-associated diseases, and preparation methods, pharmaceutical compositions, and formulations thereof. The present disclosure also disclosed the use of crystalline forms of Filgotinib hydrochloride in the prevention and/or treatment of diseases associated with JAK family. In comparison to the known crystalline forms, the crystalline forms of the present disclosure show more favorable properties in terms of formulation engineering, such as higher stability under low water activity conditions, simplicity in the preparation process and/or higher solubility.

## Description

### TECHNICAL FIELD

The present disclosure relates to the technical field of pharmaceutical crystal. In particular, it relates to novel forms of hydrochloride of a drug for treating or preventing JAK-associated diseases, preparation methods and use thereof.

### BACKGROUND

Polymorph or polymorphism is the properties of some molecules or molecular complexes. Polymorphism may result from different molecular packing. Polymorphs of a given compound may have different crystal structures and physical properties, such as solubility, stability, thermal property, mechanical property, purification ability, X-ray powder diffraction, infrared spectroscopy, Raman spectroscopy, and solid-state NMR spectroscopy, etc. One or combination of multiple characterization methods may be used to differentiate different crystalline forms of the same molecule or molecular complexes.

Novel crystalline forms (including anhydrates, hydrates and solvates) of the active pharmaceutical ingredients may offer better processing and physicochemical properties, such as bioavailability, stability, processability, purification ability. Some novel crystalline forms may serve as an intermediate crystal form to facilitate solid state transformation to a desired form. Novel polymorphs of raw materials provide more solid states in the formulation, this can improve dissolution, improving shelf life, and making it easier to process.

Studies have shown that JAK1 is essential for the inhibition of inflammatory conditions. Inhibition of JAK2, JAK3 and TYK2 is not necessary to inhibit inflammation, but is associated with some adverse reactions, for example, inhibition of JAK2 is associated with anemia, and inhibition of JAK3 is associated with immunosuppression. Filgotinib (GLPG0634) is a selective JAK1 inhibitor with IC50 of 10 nM, 28 nM, 810 nM, and 116 nM for JAK1, JAK2, JAK3, and TYK2, respectively. Clinical trials have shown that it does not cause an anemia and abnormal increase in LDL. The chemical name of the compound is: N-[5-[4-[(1,1-dioxo-1-thiomorpholin-4-yl)methyl]phenyl][1,2,4]triazole[1,5-a]pyridin -2-yl]cyclopropanecarboxamide. The chemical formula is C₂₁H₂₃N₅O₃S. The molecular weight is 425.5. The chemical structure is as shown in formula (I).

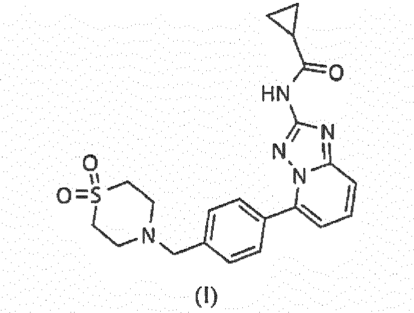

The confirmation, preparation and use of Filgotinib were disclosed in WO2010149769A1 (which is incorporated herein by reference). However, the patent does not disclose any final form information of obtained Filgotinib or its salts.

WO201511798A1 (which is incorporated herein by reference) disclosed Filgotinib monohydrochloride and its crystalline forms, including an anhydrate (Filgotinib:HCl:H₂O, 1:1:0), a trihydrate (Filgotinib:HCl:H₂O, 1:1:3), a methanol solvate (Filgotinib:HCl:MeOH, 1:1:1) and a formic acid solvate (Filgotinib:HCl:HCO₂H, 1:1:1.5). However, the solvates are less valuable than anhydrate and hydrate. The solvate form is not stable due to solvent removal after stored for a long time, and the solvate form may have poor safety. In addition, the inventor of the present disclosure finds that Filgotinib hydrochloride trihydrate with molar ratio of 1: 1: 3 in the patent is not stable under low water activity.

Therefore, there is still a need to develop novel crystalline forms of Filgotinib with desired properties such as high solubility, good flowability and/or good thermodynamic stability under low water activity. These novel crystalline forms can meet strict requirements of industrial formulation production and future drug application.

### SUMMARY

To overcome the deficiencies of prior art, the present disclosure provides novel crystalline forms of Filgotinib hydrochloride of formula (II), preparation methods and uses thereof.

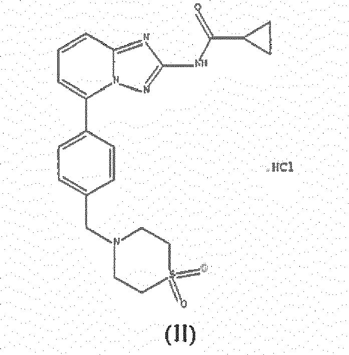

One objective of the present disclosure is to provide crystalline form A of Filgotinib hydrochloride (hereinafter referred to as Form A).

The X-ray powder diffraction pattern of Form A shows characteristic peaks at 2theta values of 7.3°±0.2°, 14.7°±0.2° and 29.6°±10.2° using Cu-Kα radiation.

In a preferred embodiment of the present disclosure, the X-ray powder diffraction pattern of Form A shows characteristic peaks at 2theta values of 7.3°±0.2°, 12.4°±0.2°, 14.7°±0.2°, 17.4°±0.2°, 20.6°±0.2° and 29.6°±0.2°.

In a further preferred embodiment, the X-ray powder diffraction pattern of Form A shows characteristic peaks at 2theta values of 7.3°±0.2°, 8.9°±0.2°, 12.4°±0.2°, 14.7°±0.2°, 16.8°±0.2°, 17.4°±0.2°, 20.6°±0.2°, 22.6°±0.2° and 29.6°±0.2°.

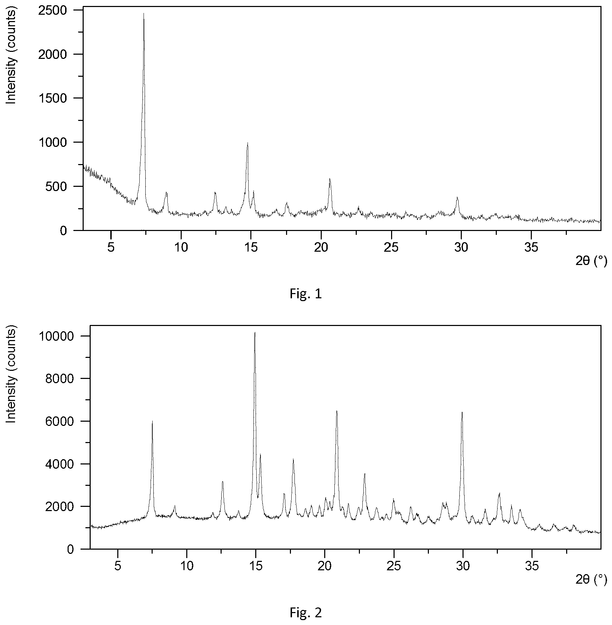

Unrestrictedly, in a specific example of the present disclosure, the X-ray powder diffraction pattern of Form A is substantially as depicted in Fig. 2.

Unrestrictedly, Form A of the present disclosure is a hydrate; furthermore, Form A has about 9%-13% weight loss when heated to 192°C.

One objective of the present disclosure is to provide preparation methods of Form A. The preparation methods comprise adding Filgotinib free base in alkyl nitriles, adding hydrochloric acid, stirring at certain temperature to crystallize, then separating and drying to obtain Form A; Or alternatively adding known crystalline form of Filgotinib·HCl·3H₂O in alkyl nitriles, stirring at certain temperature to crystallize, then separating and drying to obtain Form A.

Wherein:
Said alkyl nitriles are C₂-C₃ alkyl nitriles or combination thereof.

Preferably, said alkyl nitrile is acetonitrile, propionitrile or combination thereof.

More preferably, said alkyl nitrile is acetonitrile.

When utilizing Filgotinib free base as a starting material, the molar ratio of free base and hydrochloric acid is 1:1.0-1:1.5, preferably 1:1.0.

Preferably, the amount of said Filgotinib free base or known crystalline form of Filgotinib·HCl·3H₂O is 20-70 times of its solubility in the solvent system at operating temperature, preferably 40-50 times.

Preferably, said operating temperature is 10-40 °C, more preferably room temperature.

Preferably, said crystallization time is 36-72 hours, more preferably 48 hours.

Form A of Filgotinib hydrochloride of the present disclosure has the following advantages:
① Good stability.
② Simple preparation process and good repeatability when scaling up.
③ Good crystallinity.

In addition, compared with crystalline forms of Filgotinib·HCl·3H₂O or other solvates in prior art, Form A is more stable than those known crystalline forms under low water activity (low humidity).

One objective of the present disclosure is to provide novel crystalline form B of Filgotinib hydrochloride (hereinafter referred to as Form B).

The X-ray powder diffraction pattern of Form B shows characteristic peaks at 2theta values of 7.1°±0.2°, 13.2°±0.2° and 14.2°±0.2° using Cu-Kα radiation.

In a preferred embodiment of the present disclosure, the X-ray powder diffraction pattern of Form B shows characteristic peaks at 2theta values of 7.1°±0.2°, 13.2°±0.2°, 14.2°±0.2°, 18.8°±0.2°, 20.6°±0.2° and 28.7°±0.2°.

In a further preferred example, the X-ray powder diffraction pattern of Form B shows characteristic peaks at 2theta values of 7.1°±0.2°, 13.2°±0.2°, 14.2°±1:0.2°, 15.9°±0.2°, 16.7°±0.2°, 18.8°±0.2°, 20.6°±0.2°, 22.0°±0.2° and 28.7°±0.2°.

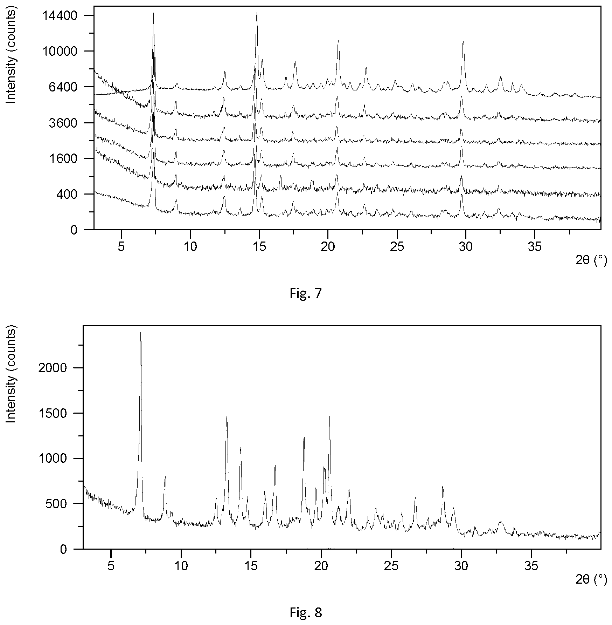

Unrestrictedly, in a specific embodiment of the present disclosure, the X-ray powder diffraction pattern of Form B is substantially as depicted in Fig. 8.

Unrestrictedly, Form B is a hydrate. Form B has about 11%-14% weight loss when heated to 200°C.

According to the objective of the present disclosure, the present disclosure provides preparation methods of Form B. The preparation methods comprise adding Filgotinib free base in ketone, adding hydrochloric acid, stirring at certain temperature to crystallize, then separating and drying to obtain Form B, or adding known crystalline form of Filgotinib·HCl·3H₂O in ketone, stirring at certain temperature to crystallize, then separating and drying to obtain Form B.

Wherein:
Preferably, said ketone is acetone.

When utilizing Filgotinib free base as the starting material, the molar ratio of free base and hydrochloric acid is 1:1.0-1:1.5, preferably 1:1.0.

Preferably, the amount of said Filgotinib free base or known crystalline form of Filgotinib·HCl·3H₂O is 20-60 times of its solubility in the solvent system at operating temperature, preferably 40-50 times.

Preferably, said operating temperature is 10-40 °C, more preferably room temperature;

Preferably, said crystallization time is 24-72 hours, more preferably 48 hours.

Form B of Filgotinib hydrochloride of the present disclosure has the following advantages:
① High solubility.
② Good crystallinity.
③ Good repeatability when scaling up and suitable for process development.

Particularly, said Form B has higher solubility compared with crystalline forms of Filgotinib·HCl·3H₂O or other solvates in prior art.

One objective of the present disclosure is to provide novel crystalline form C of Filgotinib hydrochloride (hereinafter referred to as Form C).

The X-ray powder diffraction pattern of Form C shows characteristic peaks at 2theta values of 7.0°±0.2°, 14.0°±0.2° and 20.5°±0.2° using Cu-Kα radiation.

In a preferred embodiment of the present disclosure, the X-ray powder diffraction pattern of Form C shows characteristic peaks at 2theta values of 7.0°±0.2°, 14.0°±0.2°, 16.7°±0.2°, 18.7°±0.2°, 20.5°±0.2° and 28.3°±0.2°.

In a further preferred embodiment, the X-ray powder diffraction pattern of Form C shows characteristic peaks at 2theta values of 7.0°±0.2°, 13.0°±0.2°, 14.0°±0.2°, 14.8°±0.2°, 15.8°±0.2°, 16.7°±0.2°, 18.7°±0.2°, 20.5°±0.2° and 28.3°±0.2°.

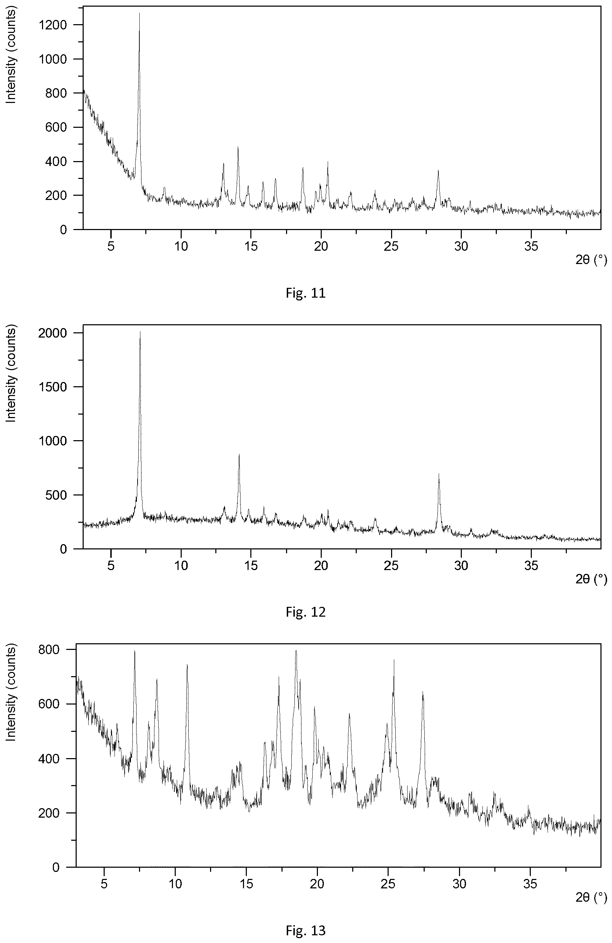

Unrestrictedly, in a specific example of the present disclosure, the X-ray powder diffraction pattern (Cu-Kα irradiation) of Form C is substantially as depicted in Fig. 11.

Unrestrictedly, Form C is a hydrate and has approximate 10%∼13% weight loss when heated to 180 °C.

According to the objective of the present disclosure, the present disclosure provides preparation methods of Form C. The preparation methods comprise adding Filgotinib free base in ketones with 4 or more carbon atoms, adding hydrochloric acid, stirring at certain temperature to crystallize, then separating and drying to obtain Form C, or adding known crystalline Form of Filgotinib·HCl·3H₂O in ketones with 4 or more carbon atoms, stirring at certain temperature to crystallize, then separating and drying to obtain Form C;

Wherein:
Said ketones with 4 or more carbon atoms are C₄-C₇ alkyl ketones or combination thereof.

Preferably, said ketones with 4 or more carbon atoms are selected from methyl ethyl ketone, methyl isobutyl ketone or combination thereof.

More preferably, said ketone with 4 or more carbon atoms is methyl ethyl ketone.

When utilizing Filgotinib free base as the starting material, the molar ratio of free base and hydrochloric acid is 1:1.0-1:1.5, preferably 1:1.0.

Preferably, said operating temperature is 10-40 °C, more preferably room temperature.

Preferably, said crystallization time is 24-72 hours, more preferably 48 hours.

Form C of Filgotinib hydrochloride of the present disclosure has the following advantages:
① High solubility.
② Good repeatability.
③ Simplicity in the preparation process.

Particularly, said Form C has higher solubility compared with crystalline forms of Filgotinib·HCl·3H₂O or other solvates in prior art.

One objective of the present disclosure is to provide novel crystalline form D of Filgotinib hydrochloride (hereinafter referred to as Form D).

The X-ray powder diffraction pattern of Form D shows characteristic peaks at 2theta values of 7.0°±0.2°, 10.7°±0.2° and 17.1°±0.2° using Cu-Kα radiation.

In a preferred embodiment of the disclosure, the X-ray powder diffraction pattern of Form D shows characteristic peaks at 2theta values of 7.0°±0.2°, 8.0°±0.2°, 8.7°±0.2°, 10.7°±0.2°, 16.2°±0.2° and 17.1°±0.2°.

In a further preferred embodiment, the X-ray powder diffraction pattern of Form D shows characteristic peaks at 2theta values of 7.0°±0.2°, 8.0°±0.2°, 8.7°±0.2°, 10.7°±0.2°, 16.2°±0.2°, 17.1°±0.2°, 18.4°±0.2°, 22.1°±0.2° and 25.3°±0.2°.

Unrestrictedly, the X-ray powder diffraction pattern (Cu-Kα irradiation) of Form D is substantially as depicted in Fig. 13.

Unrestrictedly, Form D is a hydrate and has about 11%∼14% weight loss when heated to 180 °C.

One objective of the present disclosure is to provide preparation methods of Form D. The preparation methods comprise adding Filgotinib free base in alcohols, adding hydrochloric acid, stirring at certain temperature to crystallize, then separating and drying to obtain Form D, or adding known crystalline form of Filgotini·HCl·3H₂O in alcohol, stirring at certain temperature to crystallize, then separating and drying to obtain Form D.

Wherein:
Said alcohols are C2-C5 alcohols or combination thereof.

Preferably, said alcohols are selected from ethanol, propanol, isopropanol or combination thereof.

More preferably, said alcohol is isopropanol.

When utilizing Filgotinib free base as the starting material, the molar ratio of free base: hydrochloric acid is 1:1.0-1:1.5, preferably 1:1.0.

Preferably, the amount of said Filgotinib free base or known crystalline form of Filgotinib·HCl·3H₃O is 20-70 times of its solubility in the solvent system at operating temperature, preferably 40-50 times.

Preferably, said reaction or operating temperature is 10-40 °C, more preferably room temperature.

Preferably, said crystallization time is 24-72 hours, more preferably 48 hours.

Form D of Filgotinib hydrochloride of the present disclosure has the following advantages:
① High solubility.
② Simple preparation process.
③ Low toxicity of the solvent used in preparation process;

Particularly, said Form D has higher solubility compared with crystalline forms of Filgotinib·HCl·3H₂O or other solvates in prior art.

The present disclosure has solved the problems of crystalline forms in prior art by providing novel crystalline Form A, Form B, Form C and Form D of Filgotinib hydrochloride. The novel crystalline forms have the following advantages: better stability, higher solubility and better flowability.

In the preparation methods of Form A, Form B, Form C or Form D of the present disclosure:
Said "known crystalline form of Filgotinib hydrochloride" includes the anhydrate of Filgotinib: HCl: H₂O (1:1:0), the trihydrate of Filgotinib: HCl: H₂O (1:1:3), the methanol solvate form of Filgotinib: HCl: MeOH (1:1:1) and the formic acid solvate form of Filgotinib: HCl: HCO₂H (1:1:1.5) in WO2015117981 A1.

In the present disclosure, unless otherwise specified, said "known crystalline forms of Filgotinib·HCl·3H₂O" or "crystalline form of Filgotinib·HCl·3H₂O in prior art" is specifically the crystalline form in chapter 3.2.3.2 and 3.2.3.3 etc. of WO2015117981A1 which are confirmed by table IX, Fig. 5 and Fig .11.

Said "room temperature" is 15-25 °C.

Said "stirring" is accomplished with the routine methods in this field, such as magnetic stirring or mechanical stirring; the stirring speed is 50-1800 rpm, and preferably, 300-900 rpm.

Said "separating" is accomplished by conventional methods in this filed, such as centrifuging and filtering. The operation of "centrifuging" comprises placing the sample into a centrifuge tube, then centrifuging at 10000 rpm until all the solids sink to the bottom of the tube.

Unless otherwise specified, said "drying" may be conducted at room temperature or higher temperature. The drying temperature is from room temperature to 60 °C, or to 40 °C, or to 50 °C. The drying time may be 2-48 hours, or overnight. Drying may be conducted in a fume hood, a blast drying oven or a vacuum drying oven.

In the present disclosure, "Crystal" or "Crystalline Form" is the crystal or the crystal form being identified by the X-ray diffraction pattern shown herein. The scientists in this field are able to understand that physical and chemical properties discussed herein can be characterized, wherein the experimental errors depend on the conditions of instruments, the sample preparations and the purity of samples. In particular, the scientists in this field generally know that the X-ray diffraction pattern usually may change with the change of the experimental conditions. It is necessary to point out that, the relative intensity of the X-ray diffraction pattern is likely to change with the change of the experimental conditions; therefore, the sequence of peak intensity cannot be regarded as the only or the determining factor. Moreover, generally, the experimental errors of the peak angles are 5% or less, so such errors shall be considered and generally the allowed errors are ±0.2°. In addition, due to the effect of the experimental factors including sample height, peak angles may have an overall shifting; generally, certain shifting is allowed. Hence, the scientists in this field may understand that, it is unnecessary that the X-ray diffraction pattern of a crystal form in the present disclosure should be exactly the same with X-ray diffraction patterns of the example shown herein. Any crystal forms whose X-ray diffraction patterns have the same or similar characteristic peaks should be within the scope of the present disclosure. The scientists in this field can compare the patterns shown in the present disclosure with that of an unknown crystal form in order to identify whether these two groups of patterns reflect the same or different crystal forms.

"Crystalline form" and "polymorphic form" as well as other related terms in the present disclosure refer to the solid compounds whose crystal structure is being in a special crystal form state. The difference in the physical and chemical properties of the polymorphs may be embodied in storage stability, compressibility, density, dissolution rate, etc. In extreme cases, the difference in solubility or dissolution rate may result in inefficient drugs, even developing toxicity.

In some examples, Form A, Form B, Form C and Form D of the present disclosure are pure, single forms and substantially free of any other crystalline forms. In the present disclosure, when "substantially free of" is used for describing a novel crystalline form, it means that the content of other crystalline forms in the novel crystalline form is less than 20% (w/w), specifically less than 10% (w/w), more specifically less than 5% (w/w) and further more specifically less than 1% (w/w).

It should be noted that the number and the range of number should not be narrowly understood as a value or numerical value range. It should be understood by those skilled in the art that the specific numerical value can be floated according to the specific technical environment on the basis that the spirit and principle of the present disclosure are not depart from the spirit and principle of the present disclosure. In the present disclosure, the number of floating ranges which can be expected by one of skilled in the art is represented by the term "about".

The crystalline Forms of drugs may be obtained by methods including but not limited to the following: melting recrystallization, melting cooling, recrystallization from solvent, desolvation, fast evaporation, quench cooling, slow cooling, vapor diffusion and sublimation. Sometimes, same crystals can be obtained by different methods. The crystalline Form may be tested, discovered and classified via X-ray powder diffraction (XRPD), differential scanning calorimetry (DSC), thermogravimetric analysis (TGA), optical microscopy, hygroscopicity, etc. The crystallization method of the present disclosure is slurry. Slurry means, for example, stirring the over-saturated solution (with the presence of insoluble solids) of the sample in a solvent system to crystallize, generally for 24-72 hours.

In addition, the present disclosure provides a pharmaceutical composition, which comprises a therapeutically and/or prophylactically effective amount of one or more selected forms of Form A, Form B, Form C and Form D of present disclosure, and at least one pharmaceutically acceptable excipient. Moreover, the pharmaceutical composition may also comprise other pharmaceutical acceptable crystalline Forms or the amorphous Form of Filgotinib or its salts, and such crystalline Forms include but not limited to the known crystalline Forms disclosed in WO2015117981A1. Optionally, Form A, Form B, Form C and Form D of the present disclosure can be applied as a separate active agent, or they can be applied in combination with other active agents, including other compounds which have the same or similar therapeutic activity and are determined to be safe and effective in combination with such compounds. In a particular example, Co-administration of two (or more) active agents can reduce the dosage of each active agent, so that the side effects can be reduced. The co-administration includes but is not limited to those disclosed in the specification of prior art WO2010149769A1.

The pharmaceutical composition can be developed into a certain dosage form, and is administrated by a suitable route, such as oral administration and parenteral administration (including subcutaneous, muscle, vein or skin), rectal, transdermal, nasal and vagina, and the like. The dosage form suitable for oral administration comprises tablets, capsules, granules, powder and pills, a powder, an ingot, a solution, a syrup or a suspension according to needs, and can be used for rapid release, delayed release or regulation release of active pharmaceutical ingredients. The dosage form suitable for parenteral administration comprises an aqueous or non-aqueous sterile injection solution, an emulsion or a suspension. The dosage form suitable for rectal administration comprises a suppository or an enema. The dosage form suitable for transdermal administration comprises an ointment, a cream and a patch. The dosage form suitable for nasal administration comprises an aerosol, a spray and a nose drop. The dosage form suitable for vaginal administration comprises a suppository, a plugging agent and a gel, a paste or a spray. Preferably, the crystalline forms of the present disclosure is especially suitable for preparing a tablet, a suspension, a capsule, a disintegrating tablet, an immediate release and controlled release tablet, and further preferably is a tablet, a suspension and a capsule.

The pharmaceutically acceptable excipient in the pharmaceutical composition is in the condition of a solid oral dosage form, including but not limited to: a diluent, such as starch, pregelatinized starch, lactose, powdery cellulose, microcrystalline cellulose, calcium hydrophosphate, tricalcium phosphate, mannitol, sorbitol, sugar and the like, an adhesive, such as arabic gum, guar gum, gelatin, polyvinylpyrrolidone, hydroxypropyl cellulose, hydroxypropyl methyl cellulose, polyethylene glycol and the like, a disintegrating agent, such as starch, sodium hydroxyacetate, pregelatinized starch, cross-linked povidone, cross-linked sodium carboxymethyl cellulose and colloidal silica, a lubricant, such as stearic acid, magnesium stearate, zinc stearate, sodium benzoate, sodium acetate and the like, a glidant, such as colloidal silica and the like, a compound forming agent, such as various levels of cyclodextrin and resin, a release rate control agent, such as hydroxypropyl cellulose, hydroxymethyl cellulose, hydroxypropyl methyl cellulose, ethyl cellulose, methyl cellulose, methyl methacrylate, wax and the like. Other pharmaceutically acceptable excipients, including but not limited to: a film-forming agent, a plasticizer, a coloring agent, a flavoring agent, a viscosity regulator, a preservative, an antioxidant and the like. Optionally, Apply the coating layer to the tablet, for example, providing shellac isolation coating, sugar coating or polymer coating, wherein the coating layer comprises a polymer such as hydroxypropyl methyl cellulose, polyvinyl alcohol, ethyl cellulose, methacrylic acid polymer, hydroxypropyl cellulose or starch, and can also comprise an anti-sticking agent such as silicon dioxide, talcum powder, an emulsion agent such as titanium dioxide, a colorant such as an iron oxide colorant. In the case of a liquid oral dosage form, the appropriate excipient comprises water, oils, alcohol and glycols, a preservative, a stabilizer, a coloring agent and the like. The water or the non-water sterile suspension can contain a suspending agent and a thickening agent. The excipient that is suitable for the water-based suspension comprises synthetic rubber or natural rubber can be such as arabic gum, xanthium gum, alginate, glucan, sodium carboxymethyl cellulose, methylcellulose, polyvinylpyrrolidone or gelatin. In the case of parenteral administration, the excipient of the water or non-aqueous sterile injectable solution is generally sterile water, normal saline or a glucose aqueous solution, which can contain a buffering agent, an antioxidant, a bacteriostatic agent and a solute capable of enabling the pharmaceutical composition to be combined with blood. Each excipient must be acceptable, and can be compatible with other ingredients in the formula and is harmless to a patient.

The pharmaceutical composition can be obtained by known methods in the prior art. When preparing the pharmaceutical composition, Form A, Form B, Form C or Form D of Filgotinib hydrochloride of the present disclosure is mixed with one or more pharmaceutically acceptable excipients, and mixed with one or more other active pharmaceutical ingredients. For example, the tablet, the capsule and the granule can be prepared through processes of mixing, granulating, tableting or filling capsules. The powder is prepared by mixing active pharmaceutical ingredients and excipients which are ground into a proper size. The solution and the syrup can be prepared by dissolving the active pharmaceutical ingredients in a properly flavored water or aqueous solution. The suspension can be prepared by dispersing the active pharmaceutical ingredients in pharmaceutically acceptable carriers.

What should be specially mentioned is the wet granulation process for solid dosage form. Taking the wet granulation of tablets as example, the preparation process comprises: mixing the dry solids such as the active ingredient, the bulking agent, the binder, etc. and then wetting them with a wetting agent such as water or alcohol; coagulating or granulating the wetted solids; continue the wet granulating until the required particle size of granules were uniformly obtained; after that, drying the granules. Then, mixing the drying granules with a disintegrating agent, lubricant(s), antiadherent(s), etc.; tableting the mixture in a tableting machine; and optionally, coating the tablets with suitable coating powders.

In addition, what should be specially mentioned is the oral suspension. One advantage of this administration form is that patients don't need to swallow solids, especially for elderly people, children or patients with injuries in the mouth or the throat, who may have difficulties in swallowing solids. The suspension is a two-phase system formed by dispersing solid grains into a liquid. It helps to maintain the stability of the product keeping its original solid form in water or an aqueous carrier of the suspension. The other ingredients in the oral suspension may include buffering agents, surface active agents, viscosity regulators, preservatives, antioxidants, colorants, flavoring agents and taste masking agents.

Form A, Form B, Form C and Form D of Filgotinib hydrochloride of the present disclosure have desired properties for the above dosage Forms.

In addition, the present disclosure provides use of one or more crystalline forms of Form A, Form B, Form C and Form D of Filgotinib hydrochloride for preparing drugs inhibiting JAK, especially inhibiting JAKI.

As used herein the term "JAK" is Janus kinases (JAKs) family, and it is a cytoplasmic tyrosine kinases that transduce cytokine signaling from membrane receptors to STAT transcription factors. It comprises four JAK family members: JAK1, JAK2, JAK3 and TYK2.

In addition, the present disclosure provides a method for treating or preventing diseases associated with one or more types of JAK, especially a method for treating or preventing diseases associated with JAK1. Said method comprises administering to patients in need a therapeutically and/or prophylactically effective amount of one or more of Form A, Form B, Form C or Form D, or pharmaceutical composition of one or more of Form A, Form B, Form C or Form D. Said patients include but is not limited to mammal, and said mammal can be human being.

Said diseases related to one or more JAK include but is not limited to inflammatory conditions, autoimmune diseases, proliferative diseases, transplantation rejection, diseases involving impairment of cartilage turnover, congenital cartilage malformations, and/or diseases associated with hypersecretion of IL6.

Wherein, said "inflammatory conditions" is a group of conditions, including rheumatoid arthritis, osteoarthritis, juvenile idiopathic arthritis, psoriasis, allergic airway disease (e.g. asthma, rhinitis), inflammatory bowel diseases (e.g. Crohn's disease, colitis), endotoxin-driven disease states (e.g. complications after bypass surgery or chronic endotoxin states contributing to e.g. chronic cardiac failure), and related diseases involving cartilage, such as that of the joints. Partcicularly the term is rheumatoid arthritis, osteoarthritis, allergic airway disease (e.g. asthma) and inflammatory bowel diseases.

Said "autoimmune diseases" is a group of diseases, including obstructive airways disease, such as COPD, asthma (e. g. intrinsic asthma, extrinsic asthma, dust asthma, infantily asthma), particularly chronic or inveterate asthma (for example late asthma and airway hyperreponsiveness), bronchitis, including bronchial asthma, systemic lupus erythematosus (SLE), multiple sclerosis, type I diabetes mellitus and complications associated therewith, atopic eczema (atopic dermatitis), contact dermatitis and further eczematous dermatitis, inflammatory bowel disease (e.g. Crohn's disease and ulcerative colitis), atherosclerosis and amyotrophic lateral sclerosis. Preferably, said "autoimmune diseases" is selected from COPD, asthma, systemic lupus erythematosis, type I diabetes mellitus and inflammatory bowel disease.

Said "proliferative diseases" includes cancer (e.g. uterine leiomyosarcoma or prostate cancer), myeloproliferative disorders (e.g. polycythemia vera, essential thrombocytosis and myelofibrosis), leukemia (e.g. acute myeloid leukaemia and acute lymphoblastic leukemia), multiple myeloma, psoriasis, restenosis, sclerodermitis or fibrosis. Preferably, said "proliferative diseases" is selected from cancer, leukemia, multiple myeloma and psoriasis.

Said "cancer" includes a malignant or benign growth of cells in skin or in body organs, for example but without limitation, breast, prostate, lung, kidney, pancreas, stomach or bowel. A cancer tends to infiltrate into adjacent tissue and spread (metastasise) to distant organs, for example to bone, liver, lung or the brain. Cancer of the present disclosure includes both metastatic tumour cell types, but not limited to melanoma, lymphoma, leukaemia, fibrosarcoma, rhabdomyosarcoma, and mastocytoma and types of tissue carcinoma, but not limited to colorectal cancer, prostate cancer, small cell lung cancer and non-small cell lung cancer, breast cancer, pancreatic cancer, bladder cancer, renal cancer, gastric cancer, glioblastoma, primary liver cancer, ovarian cancer, prostate cancer and uterine leiomyosarcoma.

Said "leukemia" includes neoplastic diseases of the blood and blood forming organs. In particular, leukemia is acute myeloid leukaemia (AML) and acute lymphoblastic leukemia (ALL).

Said "transplantation rejection" is the acute or chronic rejection of cells, tissue or solid organ allo- or xenografts of e.g. pancreatic islets, stem cells, bone marrow, skin, muscle, corneal tissue, neuronal tissue, heart, lung, combined heart-lung, kidney, liver, bowel, pancreas, trachea or oesophagus, or graft-versus-host diseases.

### BRIEF DESCRIPTION OF DRAWINGS

Fig.1 shows an XRPD pattern of Form A in example 1 of the present disclosure.
Fig.2 shows an XRPD pattern of Form A in example 2 of the present disclosure.
Fig.3 shows a DSC curve of Form A in example 2 of the present disclosure.
Fig.4 shows a TGA curve of Form A in example 2 of the present disclosure.
Fig.5 shows an ¹HNMR spectrum of Form A in example 2 of the present disclosure.
Fig.6 shows an XRPD overlay pattern of one-week stability of Form A of the present disclosure.
Fig.7 shows an XRPD overlay pattern of two-week stability of Form A of the present disclosure.
Fig.8 shows an XRPD pattern of Form B in example 3 of the present disclosure.
Fig.9 shows a ¹HNMR spectrum of Form B in example 3 of the present disclosure.
Fig.10 shows an XRPD pattern of Form B in example 4 of the present disclosure.
Fig.11 shows an XRPD pattern of Form C in example 5 of the present disclosure.
Fig.12 shows an XRPD pattern of Form C in example 6 of the present disclosure.
Fig.13 shows an XRPD pattern of Form D in example 7 of the present disclosure.
Fig.14 shows an XRPD pattern of Form D in example 8 of the present disclosure.
Fig.15 shows a TGA curve of Form B in example 3 of the present disclosure.
Fig.16 shows a TGA curve of Form C in example 5 of the present disclosure.
Fig.17 shows a TGA curve of Form C in example 7 of the present disclosure.

### DETAILED DESCRIPTION OF THE DISCLOSURE

The present disclosure further refers to the following examples, and said examples describe in detail the preparation and use methods of the crystalline forms of the present disclosure. It will be apparent to those skilled in the art that various modifications can be made to materials and methods without departing from the scope of the present invention.

The instruments and methods used to collect data:
X-ray powder diffraction pattern in the present disclosure is acquired by a Panalytical Empyrean X-ray powder diffractometer. The parameters of the X-ray powder diffraction method of the present disclosure are as follows:
X-ray Reflection: Cu, Kα
Kαl (Å): 1.540598. Kα2 (Å): 1.544426
Kα2 / Kα1 intensity ratio: 0.50
Voltage: 45 (kV)
Current: 40 (mA)
Divergence slit: Automatic
Scanning Mode: Continuous
Scan range: from 3.0 degree to 40.0 degree
Step size: 0.013 degree
Differential scanning calorimetry (DSC) data in the present disclosure are acquired by a TA Instruments Q200 MDSC. Its control software is Thermal Advantage, and its analysis software is Universal Analysis. Generally, 1∼10mg of sample is put into an aluminum pan (unless otherwise specified, the aluminum pan is covered). The temperature of sample was raised from room temperature to 250 °C with heating rate of 10 °C/min under the protection of dry nitrogen with flow rate of 50 mL/min, while the TA software records the heat change of the sample during the heating process. The melting point is reported based on DSC onset temperature.

Thermal gravimetric analysis (TGA) data in the present disclosure are acquired by a TA Instruments Q500 TGA. Its control software is Thermal Advantage, and its analysis software is Universal Analysis. Generally, 5∼15mg of sample is put into a platinum pan. With segmented high resolution detection, the temperature of sample was raised from room temperature to 300 °C with heating rate of 10 °C/min under the protection of dry nitrogen with flow rate of 50 mL/min, while the TA software records the weight change of the sample during the heating process.

Proton nuclear magnetic resonance spectrum data (¹HNMR) is collected from a Bruker Avance II DMX 400M HZ NMR spectrometer. 1-5 mg of sample was weighed, dissolved in 0.5mL of deuterated dimethyl sulfoxide to obtain a solution with the concentration of 2-10 mg/mL.

Unless otherwise specified, the following examples were conducted at room temperature.

Free base or known crystalline form of Filgotinib·HCl·3H₂O used in the following examples can be prepared by known method in WO2010149769A1.

### Example 1

19.9 mg of Filgotinib free base was suspended in 1.0 mL of acetonitrile and stirred at room temperature followed by adding 4 µL of hydrochloric acid (12 mol/L). The suspension was stirred at room temperature for 48 hours, then centrifuged and dried in vacuum at room temperature to obtain white crystalline solid of the hydrochloride.

The obtained crystalline solid was Form A confirmed by XRPD. The XRPD data are listed in Table 1, and the XRPD pattern is substantially as depicted in Figure 1.

**Table 1**

| 2theta | d spacing | Intensity% |
|---|---|---|
| 4.6 | 19.07 | 0.73 |
| 7.31 | 12.09 | 100.00 |
| 8.92 | 9.92 | 11.00 |
| 12.39 | 7.14 | 11.83 |
| 13.13 | 6.74 | 3.95 |
| 14.69 | 6.03 | 37.61 |
| 15.14 | 5.85 | 11.61 |
| 16.70 | 5.31 | 1.98 |
| 17.47 | 5.08 | 5.78 |
| 18.42 | 4.82 | 0.73 |
| 20.63 | 4.30 | 17.09 |
| 22.60 | 3.93 | 3.48 |
| 23.60 | 3.77 | 1.27 |
| 24.99 | 3.56 | 0.31 |
| 25.99 | 3.43 | 2.57 |
| 27.36 | 3.26 | 1.65 |
| 28.45 | 3.14 | 2.82 |
| 29.68 | 3.01 | 10.68 |
| 31.37 | 2.85 | 1.35 |
| 32.32 | 2.77 | 2.39 |
| 33.92 | 2.64 | 1.91 |

### Example 2

100.0 mg of Filgotinib free base was suspended in 5.0 mL of acetonitrile and stirred at room temperature followed by adding 20 µL of hydrochloric acid (12 mol/L). The suspension was stirred at room temperature for 48 hours, then centrifuged and dried under vacuum to obtain white crystalline solid of the hydrochloride.

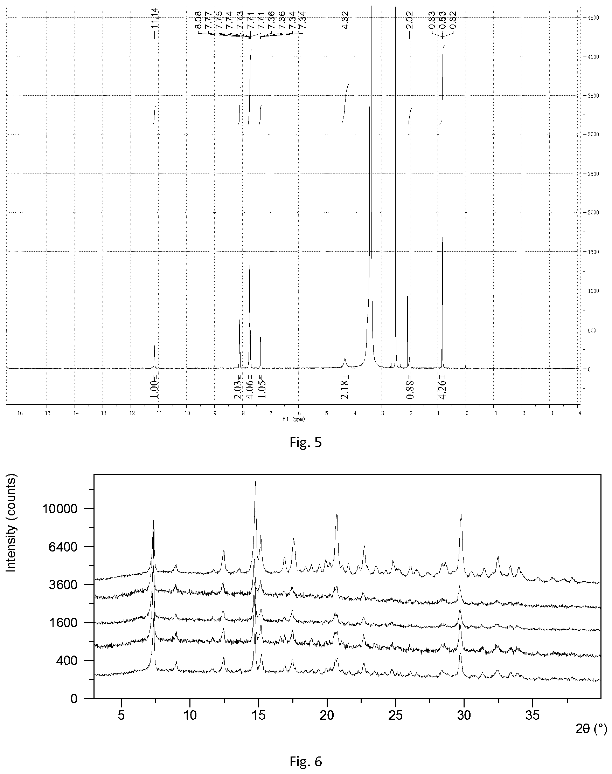

The obtained solid in this example was Form A confirmed by XRPD. The X-ray powder diffraction data is substantially as depicted in Fig. 2 and Table 2, while the ¹H NMR spectrum is shown in Fig. 5.

**Table 2**

| 2theta | d spacing | Intensity% |
|---|---|---|
| 7.31 | 12.09 | 100.00 |
| 8.90 | 9.93 | 6.21 |
| 11.64 | 7.60 | 2.0 |
| 12.33 | 7.18 | 12.42 |
| 13.38 | 6.62 | 1.16 |
| 14.70 | 6.03 | 52.86 |
| 15.05 | 5.89 | 13.01 |
| 16.75 | 5.29 | 5.24 |
| 17.40 | 5.09 | 10.38 |
| 18.37 | 4.83 | 1.55 |
| 18.75 | 4.73 | 1.72 |
| 19.28 | 4.60 | 1.92 |
| 19.82 | 4.48 | 2.43 |
| 20.66 | 4.30 | 13.64 |
| 21.42 | 4.15 | 2.07 |
| 22.13 | 4.02 | 2.32 |
| 22.66 | 3.92 | 8.76 |
| 23.38 | 3.81 | 1.77 |
| 24.70 | 3.60 | 3.36 |
| 25.98 | 3.43 | 2.34 |
| 26.55 | 3.36 | 1.26 |
| 28.35 | 3.15 | 3.12 |
| 29.70 | 3.01 | 17.45 |
| 30.44 | 2.94 | 1.40 |
| 31.43 | 2.85 | 2.03 |
| 32.32 | 2.77 | 4.45 |
| 33.25 | 2.69 | 2.79 |
| 33.83 | 2.65 | 2.11 |
| 36.32 | 2.47 | 0.66 |
| 37.76 | 2.38 | 0.99 |

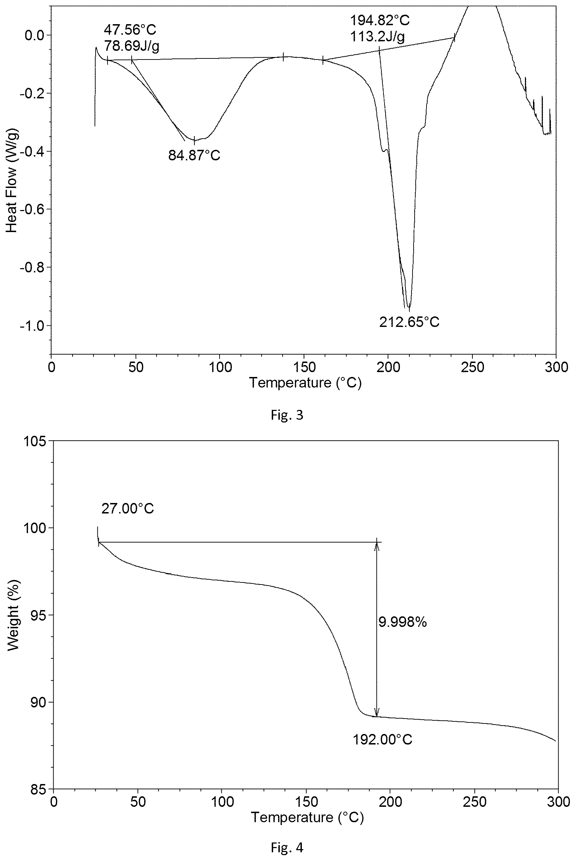

The DSC curve of this form is substantially as depicted in Fig. 3 which has two endothermic peaks. The DSC curve shows the first endothermic peak when heated to about 48 °C (onset temperature), due to the loss of crystal water, and shows the second endothermic peak when heated to 195 °C, which represents melting process.

The TGA curve of Form A is substantially as depicted in Fig. 4. It has about 10.0% weight loss when heated to 192 °C.

### The stability assessment of Form A

5 samples (10.0 mg each) of Form A obtained from example 2 was stored at 5 °C, 25 °C/60%RH, 40 °C/75%RH, 60 °C/75%RH and 80 °C for two weeks, then XRPD test was performed after one week and two weeks.

The results are depicted in Fig. 6 and Fig. 7. Fig. 6 is the XRPD overlay of one-week stability, and the patterns from top to bottom are initial Form A, and Form A after storing at 25 °C /60%RH, 40 °C/75%RH, 60 °C/75%RH, 80 °C for one week, respectively. Fig. 7 is the XRPD overlay of two-week stability, and the patterns from top to bottom are initial Form A, and Form A after storing at 25 °C/60%RH, 40 °C/75%RH, 60 °C/75%RH and 80 °C for two weeks, respectively.

The results show that Form A has good stability and it has no form change after storing at 5 °C, 25 °C/60%RH, 40 °C/75%RH, 60 °C/75%RH and 80 °C for two weeks.

### Slurry experiment for known crystalline form of Filgotinib·HCl·3H₂O in different water activities

The crystalline form of Filgotinib·HCl·3H₂O in prior art WO2015117981 A1 was utilized as the starting material, and suspended in H₂O/EtOH (0.15 water activity), H₂O/MeOH (0.2 and 0.4 water activities), H₂O/ACN (0.2 and 0.4 water activities) solvent systems for stirring. The samples were centrifuged after 48 hours, and tested by XRPD. The final solid forms were form A of the present disclosure and the results are listed in table 3.

**Table 3**

| Solvent | Water Activity | Starting Form | Final Form |
|---|---|---|---|
| H₂O/EtOH 2:98 | 0.15 | known Form of Filgotinib·HCl·3H₂O | Form A |
| H₂O/MeOH 7:93 | 0.2 | known Form of Filgotinib·HCl·3H₂O | Form A |
| H₂O/MeOH 16:84 | 0.4 | known Form of Filgotinib·HCl·3H₂O | Form A |
| H₂O/ACN 1:99 | 0.2 | known Form of Filgotinib·HCl·3H₂O | Form A |
| H₂O/ACN 2:98 | 0.4 | known Form of Filgotinib·HCl·3H₂O | Form A |

The results at low water activitiy (low relative humidity) show that Form A is more stable than the form of Filgotinib·HCl·3H₂O in prior art below 40% relative humidity.

### Example 3

20.0 mg of Filgotinib free base was suspended in 0.8 mL of acetone and stirred at room temperature followed by adding 4 µL of hydrochloric acid (12 mol/L). The suspension was stirred at room temperature for 48 hours, then centrifuged and dried in vacuum to obtain white crystalline solid of the hydrochloride.

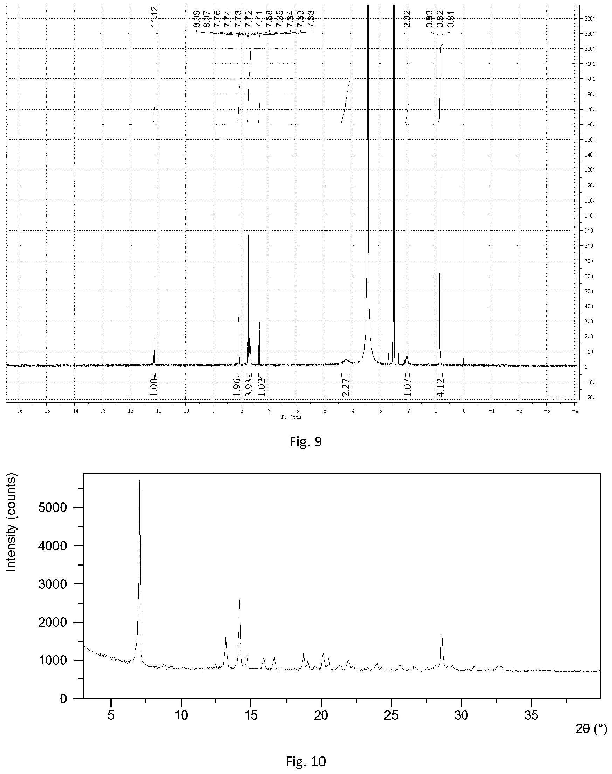

The obtained solid in this example was Form B confirmed by XRPD. The X-ray powder diffraction data is substantially as depicted in Fig. 8 and Table 4, while the ¹H NMR spectrum is shown in Fig. 9.

**Table 4**

| 2theta | d spacing | Intensity% |
|---|---|---|
| 7.10 | 12.45 | 100.00 |
| 8.85 | 9.99 | 24.69 |
| 9.29 | 9.52 | 5.14 |
| 12.50 | 7.08 | 14.90 |
| 13.21 | 6.70 | 60.08 |
| 14.22 | 6.23 | 42.62 |
| 14.72 | 6.02 | 15.33 |
| 15.95 | 5.56 | 18.79 |
| 16.71 | 5.31 | 34.68 |
| 18.75 | 4.73 | 49.61 |
| 19.60 | 4.53 | 22.51 |
| 20.21 | 4.39 | 32.80 |
| 20.60 | 4.31 | 61.13 |
| 21.18 | 4.20 | 11.98 |
| 21.94 | 4.05 | 21.58 |
| 23.32 | 3.81 | 6.00 |
| 23.85 | 3.73 | 12.10 |
| 24.40 | 3.65 | 8.32 |
| 25.18 | 3.54 | 6.07 |
| 25.73 | 3.46 | 9.89 |
| 26.70 | 3.34 | 19.11 |
| 27.62 | 3.23 | 7.19 |
| 28.66 | 3.11 | 24.98 |
| 29.40 | 3.04 | 14.22 |
| 30.97 | 2.89 | 3.39 |
| 32.73 | 2.74 | 6.61 |
| 33.80 | 2.65 | 3.15 |

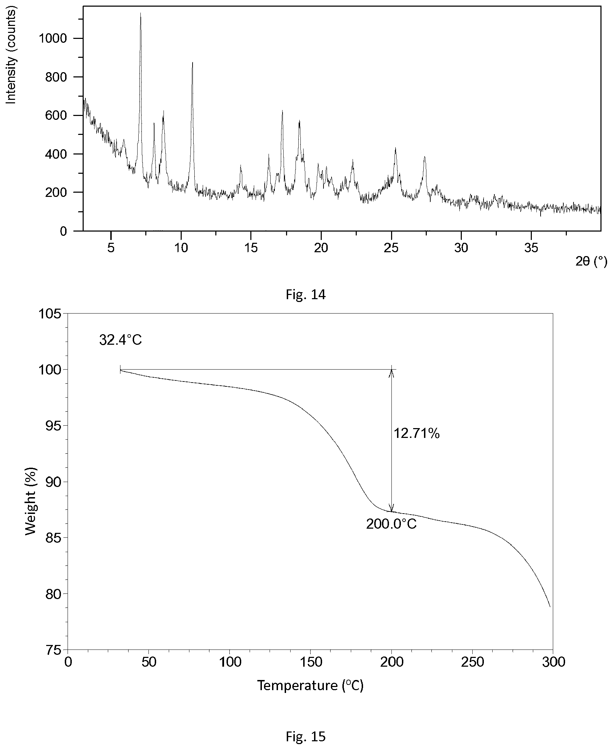

The TGA curve of this form is substantially as depicted in Fig. 15. It has approximate 12.7% weight loss when heated to 200 °C.

### Example 4

80.0 mg of Filgotinib free base was suspended in 4.0 mL of acetone and stirred at room temperature followed by adding 16 µL of hydrochloric acid (12 mol/L). The suspension was stirred at room temperature for 48 hours, then centrifuged and dried in vacuum to obtain white crystalline solid of the hydrochloride.

The obtained solid in this example was Form B confirmed by XRPD. The X-ray powder diffraction data is substantially as depicted in Fig. 10 and Table 5.

**Table 5**

| 2theta | d spacing | Intensity% |
|---|---|---|
| 4.70 | 18.80 | 3.20 |
| 7.10 | 12.45 | 100.00 |
| 8.85 | 10.00 | 7.83 |
| 13.22 | 6.69 | 28.28 |
| 14.22 | 6.23 | 34.43 |
| 14.74 | 6.01 | 11.14 |
| 15.96 | 5.55 | 12.86 |
| 16.73 | 5.30 | 13.47 |
| 18.82 | 4.72 | 20.04 |
| 19.61 | 4.53 | 7.53 |
| 20.21 | 4.39 | 16.54 |
| 20.62 | 4.31 | 17.46 |
| 22.04 | 4.03 | 10.94 |
| 23.86 | 3.73 | 5.78 |
| 25.66 | 3.47 | 4.34 |
| 26.73 | 3.33 | 4.84 |
| 27.65 | 3.23 | 3.77 |
| 28.65 | 3.12 | 25.00 |
| 29.50 | 3.03 | 3.94 |
| 30.72 | 2.91 | 1.03 |
| 32.72 | 2.74 | 3.63 |

### Example 5

30.0 mg of Filgotinib free base was suspended in 1.2 mL of methyl ethyl ketone and stirred at room temperature followed by adding 6 µL of hydrochloric acid (12 mol/L). The suspension was stirred at room temperature for 48 hours, then centrifuged and dried in vacuum to obtain white crystalline solid of the hydrochloride.

The obtained solid in this example was Form C confirmed by XRPD. The X-ray powder diffraction data is substantially as depicted in Fig. 11 and Table 6.

**Table 6**

| 2theta | d spacing | Intensity% |
|---|---|---|
| 6.99 | 12.65 | 100.00 |
| 8.80 | 10.05 | 8.38 |
| 12.98 | 6.82 | 26.23 |
| 14.03 | 6.31 | 35.93 |
| 14.76 | 6.00 | 12.12 |
| 15.83 | 5.60 | 15.54 |
| 16.71 | 5.31 | 17.46 |
| 18.66 | 4.76 | 25.03 |
| 19.59 | 4.53 | 10.16 |
| 19.92 | 4.46 | 13.69 |
| 20.46 | 4.34 | 25.28 |
| 21.11 | 4.21 | 4.18 |
| 22.09 | 4.02 | 8.55 |
| 23.79 | 3.74 | 9.46 |
| 24.50 | 3.63 | 3.99 |
| 26.52 | 3.36 | 4.83 |
| 27.25 | 3.27 | 4.81 |
| 28.34 | 3.15 | 22.82 |
| 29.15 | 3.06 | 6.13 |
| 30.58 | 2.92 | 4.37 |
| 32.24 | 2.78 | 2.64 |

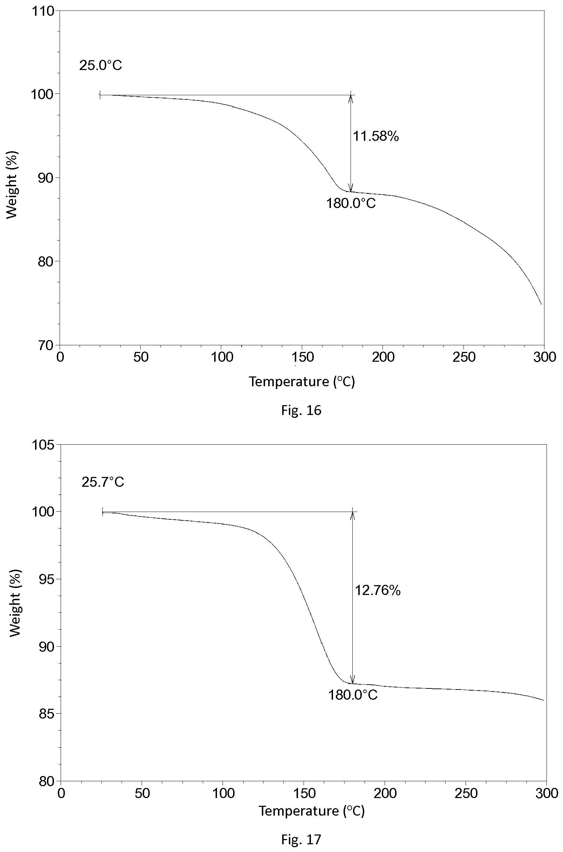

The TGA curve of this form is substantially as depicted in Fig. 16. It has approximate 11.6% weight loss when heated to 180 °C.

### Example 6

1.9 mg of the Filgotinib·HCl·3H₂O crystal in prior art was suspended in 1.5 mL of methyl ethyl ketone and stirred at room temperature for 48 hours, then centrifuged and dried in vacuum to obtain white crystalline solid of the hydrochloride.

The obtained solid in this example was Form C confirmed by XRPD. The X-ray powder diffraction data is substantially as depicted in Fig. 12 and Table 7.

**Table 7**

| 2theta | d spacing | Intensity% |
|---|---|---|
| 7.02 | 12.59 | 100.00 |
| 8.71 | 10.15 | 1.90 |
| 13.01 | 6.80 | 7.12 |
| 14.08 | 6.29 | 34.65 |
| 14.77 | 6.00 | 6.33 |
| 15.86 | 5.59 | 8.66 |
| 16.72 | 5.30 | 5.58 |
| 18.71 | 4.74 | 4.76 |
| 19.95 | 4.45 | 6.31 |
| 20.46 | 4.34 | 8.26 |
| 21.17 | 4.20 | 3.77 |
| 22.00 | 4.04 | 4.27 |
| 23.81 | 3.74 | 5.87 |
| 25.29 | 3.52 | 2.31 |
| 26.52 | 3.36 | 2.07 |
| 28.34 | 3.15 | 29.76 |
| 29.11 | 3.07 | 4.76 |
| 30.64 | 2.92 | 2.97 |
| 32.28 | 2.77 | 2.54 |
| 35.91 | 2.50 | 1.70 |
| 36.45 | 2.47 | 1.38 |

### Example 7

30.0 mg of Filgotinib free base was suspended in 1.2 mL of isopropanol and stirred at room temperature followed by adding 6 µL of hydrochloric acid (12 mol/L). The suspension was stirred at room temperature for 48 hours, then centrifuged and dried in vacuum to obtain white crystalline solid of the hydrochloride.

The obtained solid in this example was Form D confirmed by XRPD. The X-ray powder diffraction data is substantially as depicted in Fig. 13 and Table 8.

**Table 8**

| 2theta | d spacing | Intensity% |
|---|---|---|
| 3.20 | 27.58 | 78.86 |
| 5.78 | 15.28 | 57.75 |
| 7.03 | 12.58 | 97.14 |
| 8.03 | 11.02 | 58.71 |
| 8.61 | 10.27 | 83.79 |
| 10.73 | 8.25 | 89.61 |
| 12.81 | 6.91 | 20.19 |
| 13.86 | 6.39 | 29.33 |
| 14.52 | 6.10 | 34.78 |
| 16.20 | 5.47 | 47.57 |
| 16.75 | 5.29 | 45.23 |
| 17.17 | 5.16 | 82.11 |
| 18.38 | 4.83 | 100.00 |
| 18.67 | 4.75 | 84.07 |
| 19.09 | 4.65 | 33.26 |
| 19.69 | 4.51 | 66.57 |
| 20.34 | 4.37 | 44.63 |
| 20.69 | 4.29 | 37.02 |
| 22.12 | 4.02 | 62.79 |
| 24.81 | 3.59 | 58.50 |
| 25.27 | 3.53 | 87.94 |
| 27.33 | 3.26 | 71.64 |
| 28.07 | 3.18 | 24.97 |
| 30.62 | 2.92 | 18.03 |
| 32.37 | 2.77 | 16.25 |
| 34.74 | 2.58 | 7.39 |

The TGA curve of this form is substantially as depicted in Fig. 17. It has approximate 12.8% weight loss when heated to 180 °C.

### Example 8

80.0 mg of Filgotinib free base was suspended in 4.0 mL of isopropanol and stirred at room temperature followed by adding 16 µL of hydrochloric acid (12 mol/L). The suspension was stirred at room temperature for 48 hours, then centrifuged and dried in vacuum to obtain white crystalline solid of the hydrochloride.

The obtained solid in this example was Form D confirmed by XRPD. The X-ray powder diffraction data is substantially as depicted in Fig. 14 and Table 9.

**Table 9**

| 2theta | d spacing | Intensity% |
|---|---|---|
| 5.84 | 15.13 | 20.65 |
| 7.05 | 12.55 | 100.00 |
| 8.03 | 11.02 | 35.43 |
| 8.58 | 10.30 | 67.20 |
| 10.73 | 8.25 | 85.74 |
| 14.46 | 6.13 | 31.04 |
| 16.21 | 5.47 | 33.68 |
| 17.11 | 5.18 | 87.90 |
| 18.13 | 4.89 | 54.39 |
| 18.37 | 4.83 | 96.42 |
| 18.65 | 4.76 | 86.86 |
| 19.67 | 4.51 | 49.84 |
| 19.96 | 4.45 | 37.49 |
| 20.51 | 4.33 | 27.67 |
| 22.12 | 4.02 | 62.41 |
| 24.74 | 3.60 | 35.60 |
| 25.25 | 3.53 | 70.70 |
| 27.30 | 3.27 | 75.94 |
| 28.11 | 3.17 | 23.84 |
| 28.53 | 3.13 | 12.63 |
| 30.67 | 2.92 | 12.58 |
| 32.20 | 2.78 | 18.34 |

### Example 9 Solubility assessment

The obtained Form A (example 2), Form B (example 4), Form C (example 5), Form D (example 8) and the form of Filgotinib·HCl·3H₂O in WO2015117981A1 (reference form) were added into simulated gastric fluid (SGF) and water to obtain saturated solutions. The concertration of the saturated solutions was measured through HPLC after 1 hour and 4 hours. The experimental results are listed in Table 10 and Table 11.

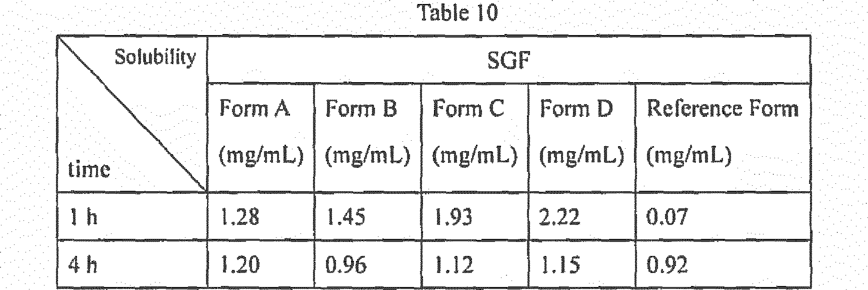

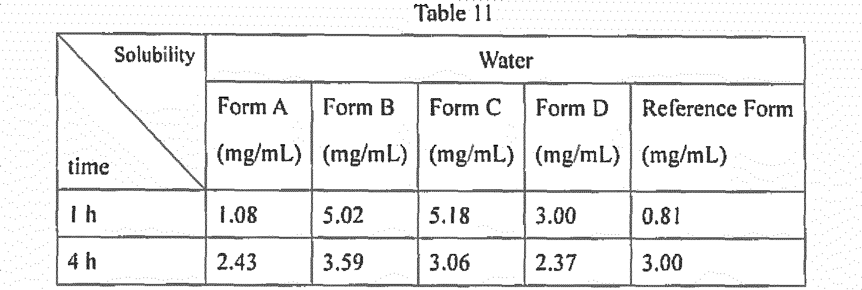

The results of the experiments show that Form A, Form B, Form C and Form D in the present disclosure have higher solubility than the reference form after being stored for I hour, 4 hours and 24 hours in the saturated solutions. Higher solubility can promote the absorption of the drugs in organisms, and improve the efficacy.

### Example 10 Particle size comparison experiment

The particle size distributions of Form A, Form B, Form C, Form D in the present disclosure and the form of Filgotinib·HCl·3H₂O in WO2015117981 A1 (reference form) were measured by Microtrac S3500 laser particle size analyzer (Microtrac S3500 was equiped with a sample delivery controller(SDC) system).

Wet method was used and Isopar G was used as the dispersion medium. The parameters of the laser particle size analyzer are listed in Table 12.

**Table 12**

| | |
|---|---|
| Size distribution: Volume | Run Time: 10 s |
| Dispersion medium: Isopar G | Particle coordinates: Standard |
| Run Number: Average of 3 runs | Fluid refractive index: 1.42 |
| Particle Transparency:: Trans | Residuals: Enabled |
| Particle refractive index: 1.5 | Flow rate: 60 * |
| Particle shape: Irregularity | Filtration: Enabled |
| Ultrasonication power: 30 W | Ultrasonication time: 30 s |

| | |
|---|---|
| *: Flow rate 60 represents 60% of 65 mL/s. | |

The particle size distributions of obtained Form A (example 2), Form B (example 4), Form C (example 5), Form D (example 8) and the reference form were measured. The results are listed in Table 13.

**Table13**

| Form | MV (µm) | D10(µm) | D50 (µm) | D90 (µm) |
|---|---|---|---|---|
| Reference form | 7.24 | 2.95 | 6.29 | 12.35 |
| Form A | 24.64 | 1.72 | 8.93 | 62.08 |
| Form B | 44.66 | 2.53 | 20.30 | 110.4 |
| Form C | 29.92 | 1.87 | 13.57 | 66.16 |
| Form D | 41.24 | 5.77 | 25.92 | 82.42 |

| | | | | |
|---|---|---|---|---|
| MV: Average particle size calculated by volume. D10: The D10 describes the diameter where 10% of the distribution has a smaller particle size. D50: The D50 describes the diameter where 50% of the distribution has a smaller particle size. The median is also called D50. D90: The D90 describes the diameter where 90% of the distribution has a smaller particle size. | | | | |

The MV of reference form is 7.24 µm. In the present disclosure, the MV of Form A is 24.64 µm, the MV of Form B is 44.66 µm, the MV of Form C is 29.92 µm, and the MV of Form D is 41.24 µm. All of them are larger than the reference form. Generally, product with larger particle size can be easily separated by filtration and thus filtration time was saved.. Meanwhile, drug stability will be increased due to larger particle size. If the product has larger particle size the downstream process will be easy.

Those skilled in the art may understand that, under the guidance of this description, some modifications or changes may be made to the present invention. These modifications and changes should be within the scope of the invention as defined in the claims.

## Claims

1. A crystalline Form B of Filgotinib hydrochloride with the following chemical structure, wherein the X-ray powder diffraction pattern shows characteristic peaks at 2theta values of 7.1°±0.2°, 13.2°±0.2° and 14.2°±0.2° using Cu-Kα radiation.

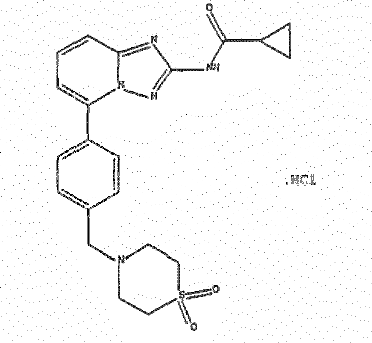

2. The crystalline Form B according to claim 2, wherein the X-ray powder diffraction pattern shows characteristic peaks at 2theta values of 7.1°±0.2°, 13.2°±0.2°, 14.2°±0,2°, 18.8°±0.2°, 20.6°±0.2° and 28.7°±0.2°.

3. The crystalline Form B according to claim 3, wherein the X-ray powder diffraction pattern shows characteristic peaks at 2theta values of 7.1°±0.2°, 13.2°±0.2°, 14.2°±0.2°, 15.9°±0.2°, 16.7°±0.2°, 18.8°±0.2°, 20.6°±0.2°, 22.0°±0.2° and 28.7°±0.2°.

4. A preparation method of crystalline Form B according to any one of claims 1-3, wherein the method comprises:
Adding Filgotinib free base in acetone, adding hydrochloric acid, stirring at certain temperature to crystallize, then separating and drying to obtain Form B;
Or alternatively adding known crystalline form of Filgotinib·HCl·3H₂O in acetone, stirring at certain temperature to crystallize, then separating and drying to obtain Form B.

5. A crystalline Form A of Filgotinib hydrochloride with the following chemical structure, wherein the X-ray powder diffraction pattern shows characteristic peaks at 2theta values of 7.3°±0.2°, 14.7°±0.2° and 29.6°±0.2° using Cu-Kα radiation.

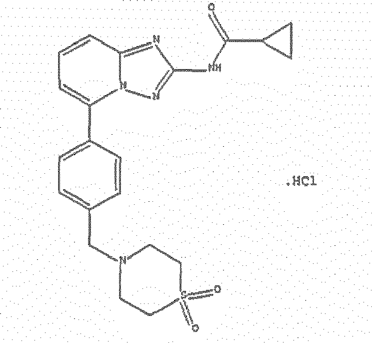

6. The crystalline Form A according to claim 5, wherein the X-ray powder diffraction pattern shows characteristic peaks at 2theta values of 7.3°±0.2°, 12.4°±0.2°, 14.7°±0.2°, 17.4°±0.2°, 20.6°±0.2° and 29.6°±0.2°.

7. The crystalline Form A according to claim 6, wherein the X-ray powder diffraction pattern shows characteristic peaks at 2theta values of 7.3°±0.2°, 8.9°±0.2°, 12.4°±0.2°, 14.7°±0.2°, 16.8°±0.2°, 17.4°±0.2°, 20.6°±0.2°, 22.6°±0.2° and 29.6°±0.2°.

8. A preparation method of crystalline Form A according to any one of claims 5-7, wherein the method comprises:
Adding Filgotinib free base in alkyl nitriles, adding hydrochloric acid, stirring at certain temperature to crystallize, then separating and drying to obtain Form A;
Or alternatively adding known crystalline form of Filgatinib·HCl·3H₂O in alkyl nitriles, stirring at certain temperature to crystallize, then separating and drying to obtain Form A.

9. The preparation method of crystalline Form A according to claim 8, wherein said alkyl nitriles are C₂∼C₃ alkyl nitriles or combination thereof, preferably acetonitrile, propionitrile or combination thereof.

10. The preparation method of crystalline Form A according to claim 9, wherein said alkyl nitrile is acetonitrile.

11. A crystalline Form C of Filgotinib hydrochloride with the following chemical structure, wherein the X-ray powder diffraction pattern shows characteristic peaks at 2theta values of 7.0°±0.2°, 14.0°±0.2° and 20.5°±0.2° using Cu-Kα radiation.

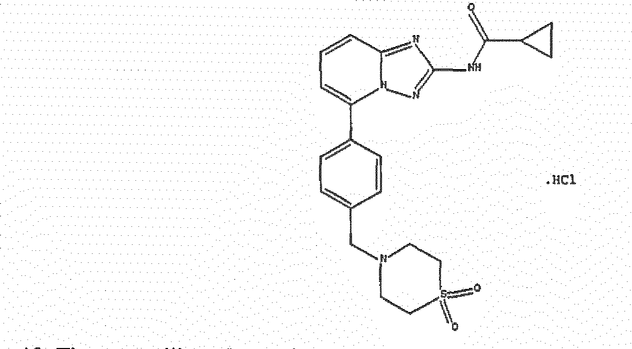

12. The crystalline Form C according to claim 11, wherein the X-ray powder diffraction pattern shows characteristic peaks at 2theta values of 7.0°±0.2°, 14.0°±0.2°, 16.7°±0.2°, 18.7°±0.2°, 20.5°±0.2° and 28.3°±0.2°.

13. The crystalline Form C according to claim 12, wherein the X-ray powder diffraction pattern shows characteristic peaks at 2theta values of 7.0°±0.2°, 13.0°±0.2°, 14.0°±0.2°, 14.8°±0.2°, 15.8°±0.2°, 16.7°±0.2°, 18.7°±0.2°, 20.5°±0.2° and 28.3°±0.2°.

14. A preparation method of crystalline Form C according to any one of claims 11-13, wherein the method comprises:
Adding Filgotinib free base in ketones with 4 or more carbon atoms, adding hydrochloric acid, stirring at certain temperature to crystallize, then separating and drying to obtain Form C;
Or alternatively adding known crystalline form of Filgotinib·HCl·3H₂O in ketones with 4 or more carbon atoms, stirring at certain temperature to crystallize, then separating and drying to obtain Form C.

15. The preparation method of crystalline Form C according to claim 14, wherein said ketones with 4 or more carbon atoms are C₄∼C₇ alkyl ketones or combination thereof, preferably methyl ethyl ketone, methyl isobutyl ketone or combination thereof.

16. The preparation method of crystalline Form C according to claim 15, wherein said ketone with 4 or more carbon atoms is methyl ethyl ketone.

17. A crystalline Form D of Filgotinib hydrochloride with the following chemical structure, wherein the X-ray powder diffraction pattern shows characteristic peaks at 2theta values of 7.0°±0.2°, 10.7°±0.2° and 17.1°±0.2° using Cu-Kα radiation.

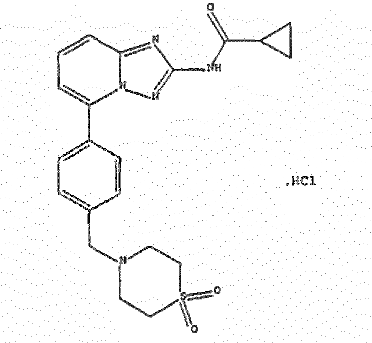

18. The crystalline Form D according to claim 17, wherein the X-ray powder diffraction pattern shows characteristic peaks at 2theta values of 7.0°±0.2°, 8.0°±0.2°, 8.7°±0.2°, 10.7°±0.2°, 16.2°±0.2° and 17.1°±0.2°.

19. The crystalline Form D according to claim 18, wherein the X-ray powder diffraction pattern shows characteristic peaks at 2theta values of 7.0°±0.2°, 8.0°±0.2°, 8.7°±0.2°, 10.7°±0.2°, 16.2°±0.2°, 17.1°±0.2°, 18.4°±0.2°, 22.1°±0.2° and 25.3°±0.2°.

20. A preparation method of crystalline Form D according to any one of claims 17-19, wherein the method comprises:
Adding Filgotinib free base in alcohols, adding hydrochloric acid, stirring at certain temperature to crystallize, then separating and drying to obtain Form D;
Or alternatively adding known crystalline form of Filgotinib·HCl·3H₂O in alcohol, stirring at certain temperature to crystallize, then separating and drying to obtain Form D.

21. The preparation method of crystalline Form D according to claim 20, wherein said alcohols are C₂-C₅ alcohols or combination thereof, preferably ethanol, propanol, isopropanol or combination thereof.

22. The preparation method of crystalline Form D according to claim 21, wherein said alcohol is isopropanol.

23. A pharmaceutical composition, which comprises a therapeutically and/or prophylactically effective amount of one or more crystalline forms selected from crystalline Form A according to any one of claims 1-3, crystalline Form B according to any one of claims 7-9, crystalline Form C according to any one of claims 11-13, crystalline Form D according to any one of claims 17-19 and at least one pharmaceutically acceptable carrier or excipient.

24. A dosage form, which comprises a therapeutically and/or prophylactically effective amount of one or more crystalline forms selected from crystalline Form A according to any one of claims 1-3, crystalline Form B according to any one of claims 7-9, crystalline Form C according to any one of claims 11-13, and crystalline Form D according to any one of claims 17-19.

25. The dosage form according to claim 24, wherein said dosage form is a tablet, a capsule, a suspension formulation, a disintegrating tablet, or an immediate release and controlled release tablet.

26. Use of crystalline Form A according to any one of claims 1-3, crystalline Form B according to any one of claims 7-9, crystalline Form C according to any one of claims 11-13, and crystalline Form D according to any one of claims 17-19 for preparing drugs inhibiting one or more types of JAK.

27. The use of crystalline Form A according to any one of claims 1-3, crystalline Form B according to any one of claims 7-9, crystalline Form C according to any one of claims 11-13, and crystalline Form D according to any one of claims 17-19 for preparing drugs treating inflammatory conditions, autoimmune diseases, proliferative diseases, transplantation rejection, diseases involving impairment of cartilage turnover, congenital cartilage malformations, and/or diseases associated with hypersecretion of IL6.

28. A method for treating or preventing diseases associated with one or more types of JAK, wherein said method comprises administering to patients in need a therapeutically and/or prophylactically effective amount of crystalline Form A according to any one of claims 1-3, crystalline Form B according to any one of claims 7-9, crystalline Form C according to any one of claims 11-13, crystalline Form D according to any one of claims 17-19, or the pharmaceutical composition according to claim 23, or the dosage form according to claim 24 or 25.
